# EUROPÄISCHE PATENTANMELDUNG

(11) **EP 0 888 788 A1**
(43) Veröffentlichungstag der Anmeldung: **07.01.1999**
(21) Anmeldenummer: 98115138.4
(22) Anmeldetag: 20.10.1993
(51) Int. Cl.: A61M 1/00, E05C 19/02, F04B 43/12

(54) **Einschubelement für eine ophthalmologische Einrichtung**

(30) Priorität: 06.11.1992 CH 3448/92; 14.10.1993 CH 3096/93
(62) Teilanmeldung aus: 93116916.3
(71) Anmelder: GRIESHABER & CO. AG SCHAFFHAUSEN, CH-8203 Schaffhausen (CH)
(72) Erfinder: Grieshaber, Hans R., 8200 Schaffhausen (CH); Demmerle, Rudolf, 8200 Schaffhausen (CH); Vogel, Urs, 8200 Schaffhausen (CH)
(74) Vertreter: Althoff, Gerhard

(57) **Zusammenfassung**

Die Erfindung bezieht sich auf ein Einschubelement (30) für eine mit einem Aspirations- und Irrigationssystem versehene ophthalmologische Einrichtung (100) zur Verwendung bei mikrochirurgischen Eingriffen am Auge eines Lebewesens.

Das als Schlauchträger ausgebildete Einschubelement (30) wird in ein Gehäuse (20) eingeführt und darin durch eine Verriegelungsmechanik gehalten und ist dabei derart in dem Gehäuse angeordnet, dass eine erste Schlauchleitung (11) für die Irrigation mit einem Quetschelement wirkverbunden und eine zweite Schlauchleitung für die Aspiration mit einem um eine im wesentlichen quer zur Einschubrichtung orientierte Achse drehbar angetriebenen Pumpenrad peristaltisch zusammenwirkend in Einriff bringbar ist.

## Beschreibung

Die Erfindung bezieht sich auf ein Einschubelement mit einer ersten und einer zweiten Schlauchleitung, welches in das Gehäuse einer ophthalmologischen Einrichtung einschiebbar sowie durch eine Verriegelungsmechanik derart gehalten und angeordnet ist, dass die erste Schlauchleitung mit einem in dem Gehäuse angeordneten Quetschelement wirkverbunden und die zweite Schlauchleitung im Bereich einer Kurvenbahn mit einem ebenfalls in dem Gehäuse angeordneten und um eine Achse drehbar angetriebenen Pumpenrad peristaltisch zusammenwirkend in Eingriff bringbar ist.

Aus der US-A 4,904,168 ist ein im wesentlichen als ebene Platte ausgebildetes Trägerelement mit zwei in entsprechend ausgebildeten Kanälen angeordneten Schlauchleitungen für ein ophthalmologisches Aspirations- und Irrigationssystem bekannt, wobei das plattenförmige Trägerelement in den Innenraum eines Gehäuses einsetzbar sowie durch zwei darin angeordnete und seitlich mit dem Trägerelement in Eingriff bringbare Verriegelungshebel fixiert und durch einen an das Gehäuse anschraubbaren Deckel gehalten ist, derart, dass ein an der einen Seite des Deckels gelagertes Pumpenrad von einem an der anderen Seite des Deckels gelagerten Antriebsaggregat um eine Achse rotierend antreibbar ist und dabei die am äusseren Umfang des Pumpenrades gelagerten Rollen mit der Aspirations-Schlauchleitung peristaltisch zusammenwirkend in Eingriff bringbar sind.

Aus der WO-A 86/06964 ist in Verbindung mit einer Einrichtung zur Durchführung mikrochirurgischer Eingriffe am Auge eines Lebewesens ein mit zwei Gehäuseteilen versehenes und zur Aufnahme einer Aspirations- und einer Irrigationsleitung ausgebildetes Trägerelement bekannt, bei welchem die beiden Gehäuseteile mit den darin angeordneten Schlauchleitungen zu einem kassettenförmigen Einschubelement zusammensteckbar sind, welches an der einen Längsseite eine erste Ausnehmung zur Aufnahme eines mit der Aspirationsleitung peristaltisch zusammenwirkend in Eingriff bringbaren Pumpenrades sowie eine zweite Ausnehmung aufweist, die zum Einführen eines mit der Irrigationsleitung in Eingriff bringbaren Schliessnockens ausgebildet ist.

Ein weiteres, mit zwei zusammensteckbaren Gehäuseteilen versehenes und im wesentlichen kassettenförmig ausgebildetes Trägerelement ist aus der EP-A 0 362 822 bekannt, wobei das mit einer im Innenraum desselben angeordneten Irrigations- und einer Aspirationsleitung versehene Trägerelement stirnseitig an eine ophthalmologische Einrichtung angeordnet und durch ein schwenkbar daran gelagertes Verschlusselement in der Funktionsstellung gehalten wird, in welcher ein elektromotorisch angetriebenes Pumpenrad mittels am äusseren Umfang daran verteilt zueinander angeordneter Rollen peristaltisch mit der Aspirationsleitung zusammenwirkend in Eingriff bringbar ist.

Aus den Druckschriften US-A 4,963,131 und EP-A 0 293 081 sind weitere und jeweils zur Aufnahme einer Irrigationsleitung sowie einer Aspirationsleitung ausgebildete Trägerelemente bekannt, welche im wesentlichen mit zwei zusammensteckbaren Gehäuseteilen kassettenförmig ausgebildet sind.

Der Erfindung liegt die Aufgabe zugrunde, ein Einschubelement gemäss dem Oberbegriff des Patentanspruchs 1 dahingehend zu verbessern und so auszubilden, dass die für die Aspiration und Irrigation vorgesehenen Schlauchleitungen beim Einführen des Einschubelements exakt und betriebssicher mit den in einzelnen Ebenen im Gehäuse angeordneten Funktionselementen in Eingriff bringbar sind.

Gelöst wird diese Aufgabe gemäss der Erfindung durch ein an einem Griffstück in Einschubrichtung orientiertes erstes Tragteil mit daran angeordnetem ersten Kanal für die erste Schlauchleitung sowie ein quer zur Einschubrichtung im Abstand zu dem ersten Tragteil angeordnetes und im wesentlichen als eine senkrecht zur Einschubrichtung orientierte Bodenplatte ausgebildetes zweites Tragteil, welches an der dem ersten Tragteil zugewandten Seite mit der Verriegelungsmechanik in Eingriff bringbare Mittel aufweist und an der der ersten Schlauchleitung abgewandten Seite mit einem zweiten Kanal für die zweite Schlauchleitung versehen ist, wobei die zweite Schlauchleitung an einer stirnseitig an dem plattenförmigen Tragteil angeordneten und analog der Kurvenbahn ausgebildeten Bogenleiste anliegend mit dem Pumpenrad in Eingriff bringbar ist.

Weitere Merkmale dar Erfindung ergeben sich aus der folgenden Beschreibung in Verbindung mit der Zeichnung und den Patentansprüchen.

Die Erfindung wird nachstehend anhand der Zeichnung beschrieben. Es zeigt:
**Fig.1** eine in perspektivischer Ansicht dargestellte ophthalmologische Einrichtung mit einem herausgezogen dargestellten und als Schlauchträger ausgebildeten Einschubelement;
**Fig.2** ein in grösserem Massstab und im Schnitt dargestelltes Teilstück eines Trägerelements mit einer in einer Funktionsebene angeordneten Einzugsmechanik für das teilweise herausgezogen dargestellte Einschubelement;
**Fig.3** das in grösserem Massstab und im Schnitt dargestellte Teilstück des Trägerelements mit einer in einer funktionsebene angeordneten und mit dem Einschubelement im Eingriff stehenden Pumpeneinheit;
**Fig.4** das in grösserem Massstab und gemäss Pfeilrichtung IV in Fig.1 in Seitenansicht dargestellte Einschubelement mit einer ersten Schlauchträgerebene;
**Fig.5** das in grösserem Massstab und gemäss Pfeilrichtung V in Fig.1 in Seitenansicht dargestellte Einschubelement mit einer zweiten Schlauchträgerebene; und
**Fig.6** das gemäss der Linie VI-VI in Fig.5 im Schnitt dargestellte Einschubelement.

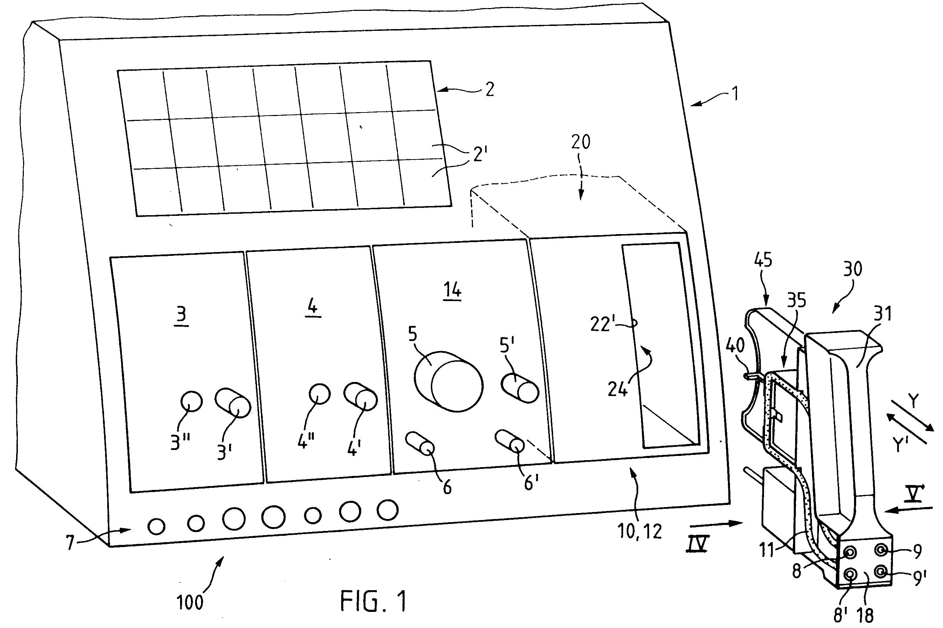

Fig.1 zeigt eine in perspektivischer Ansicht dargestellte und in der Gesamtheit mit 100 bezeichnete ophthalmologische Einrichtung zur Verwendung bei mikrochirurgischen Eingriffen am Auge eines Lebewesens. Die Einrichtung 100 umfasst mehrere, in einem Gehäuse 1 angeordnete und für den jeweiligen operativen Eingriff ausgebildete Funktionseinheiten 3,4,10,12 und 14. Die einzelnen Funktionseinheiten 3,4,10,12 und 14 sind vorzugsweise als nicht näher dargestellte, auswechselbare Einschübe ausgebildet und von der Frontseite in das Gehäuse 1 einschiebbar. Die beiden ersten Funktionseinheiten 3 und 4 sind mit nicht dargestellten Mitteln versehen, mittels welcher beim operativen Eingriff der Augeninnenraum beleuchtet werden kann. Hierzu wird ein nicht dargestellten, an dem einen Ende mit einer Lichtquelle und an dem anderen Ende mit einem Anschlussteil versehenes Lichtleiterkabel an die entsprechend ausgebildeten Buchsen 3'' und/oder 4'' der Funktionseinheiten 3 und/oder 4 angeschlossen. Die für den operativen Eingriff erforderliche Lichtstärke kann über einen Drehknopf 3' und/oder 4' stufenlos eingestellt und reguliert werden. Weiterhin erkennt man in Fig.1 eine an der Stirnseite des Gehäuses 1 mit mehreren Anschlüssen versehene Steckerleiste 7, welche zum Anschluss zusätzlicher, nicht näher dargestellter Operationsinstrumente oder dergleichen vorgesehen ist.

An der Frontseite des Gehäuses 1 ist oberhalb der einzelnen Funktionseinheiten ein Bildfeld 2 angeordnet, welches beispielsweise in einzelne Anzeige- und Bedienungsfelder 2' mit LCD-Anzeige (Liquid Crystal Display) unterteilt ist. Das einzelne Anzeige- und Bedienungsfeld 2' dient als programmabhängig gesteuerter Informationsaustausch für den Benutzer der gesamten ophthalmologischen Einrichtung 100. Die einzelnen Anzeige- und Bedienungsfelder 2' sind durch geringfügigen Fingerdruck für die jeweilige Programmwahl betätigbar, wobei die angewählte Anzeige oder angewählten Anzeigen in bezug auf die nicht betätigten Anzeigen dann beleuchtet sind.

In der nachstehenden Beschreibung wird die erste Funktionseinheit 10 als Irrigationseinheit 10, die zweite Funktionseinheit 12 als Aspirationseinheit 12 bezeichnet. Die dritte Funktionseinheit 14 wird als Druckeinheit 14 bezeichnet. Die beiden Einheiten 10 und 12 sind über nicht dargestellte Versorgungs- und Entsorgungsleitungen miteinander verbunden und bilden zusammen ein nicht näher dargestelltes Aspirations- und Irrigationssystem.

Die Druckeinheit 14 hat mindestens einen Stutzen 6 zum Anschliessen einer Leitung sowie einen Einstellknopf 5 zum Regulieren des intraokularen Druckes (IOP) beim operativen Eingriff. Der Druckeinheit 14 kann eine nicht näher dargestellte Visko-Injektionseinrichtung zugeordnet werden, welche einen Stutzen 6' zum Anschliessen einer nicht dargestellten Leitung sowie einen Einstellknopf 5' zum Regulieren aufweist.

Die einzelnen, in Fig.1 nicht näher dargestellten Funktionselemente der Irrigationseinheit 10 sowie der Aspirationseinheit 12 stehen über ein in Fig.1 schematisch dargestelltes und in das Gehäuse 1 der Einrichtung 100 hineinschiebbares Einschubelement 30 miteinander in Wirkverbindung. An der Stirnseite des Einschubelements 30 sind im Abstand zueinander angeordnete Anschlussstutzen 8,8' und 9,9' vorgesehen, an welche die einzelnen, nicht dargestellten Versorgungs- und Entsorgungsleitungen des ebenfalls nicht dargestellten Aspirations- und Irrigationssystems angeschlossen werden.

Zur Aufnahme des Einschubelementes 30 ist in dem Gehäuse 1 eine entsprechend ausgebildete und mit einer Öffnung 22' versehene Kammer 24 angeordnet. Vorzugsweise ist jedoch ein in das Gehäuse 1 einsetzbares Trägerelement 20 vorgesehen, welches die Kammer 24 aufweist, in welche das Einschubelement 30 eingeführt und wieder herausgezogen worden kann.

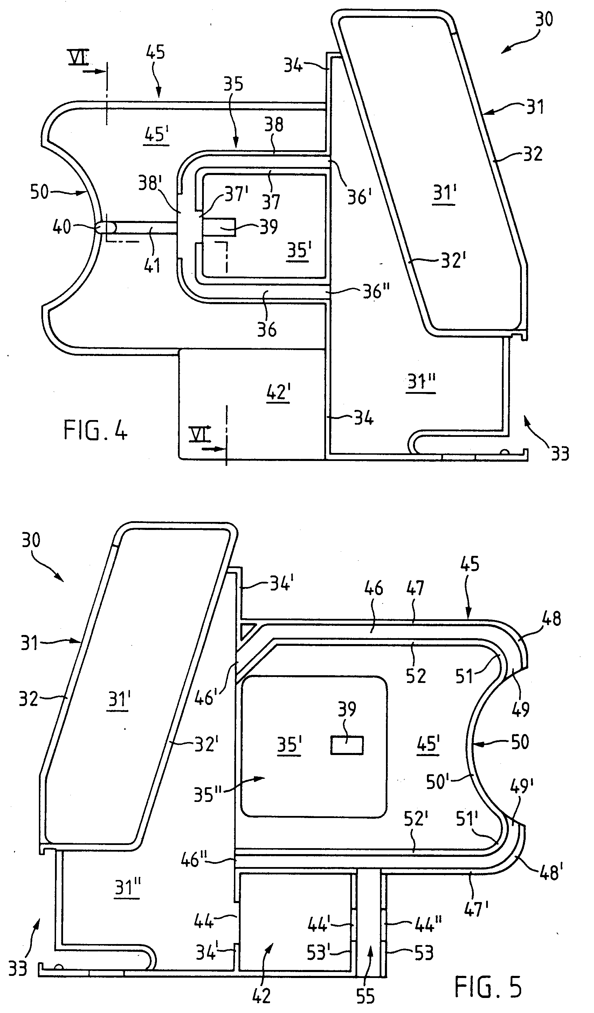

Wie in Fig.1 schematisch dargestellt, kann das als Schlauchträger ausgebildete Einschubelement 30 gemäss Pfeilrichtung Y aus dem im Gehäuse 1 angeordneten und schematisch dargestellten Trägerelement 20 herausgezogen und in Pfeilrichtung Y' wieder in die Kammer 24 des Trägerelements 20 hineingeschoben werden. Bei dem in Fig.1 schematisch dargestellten Einschubelement 30 erkennt man ein Griffstück 31, eine daran stirnseitig angeordnete Platte 18 mit den daran im Abstand zueinander angeordneten Anschlussstutzen 8,8' und 9,9' für die nicht dargestellten Versorgungs- und Entsorgungsleitungen. Das Einschubelement 30 hat ein erstes Tragteil 35 und ein zweites Tragteil 45 sowie einen Rastnocken 40, welcher auf der dem ersten Tragteil 35 zugewandten Seite des zweiten Tragteils 45 angeordnet ist. Der konstruktive Aufbau des Einschubelements 30 wird später in Verbindung alt den Figuren 4 bis 6 im einzelnen beschrieben.

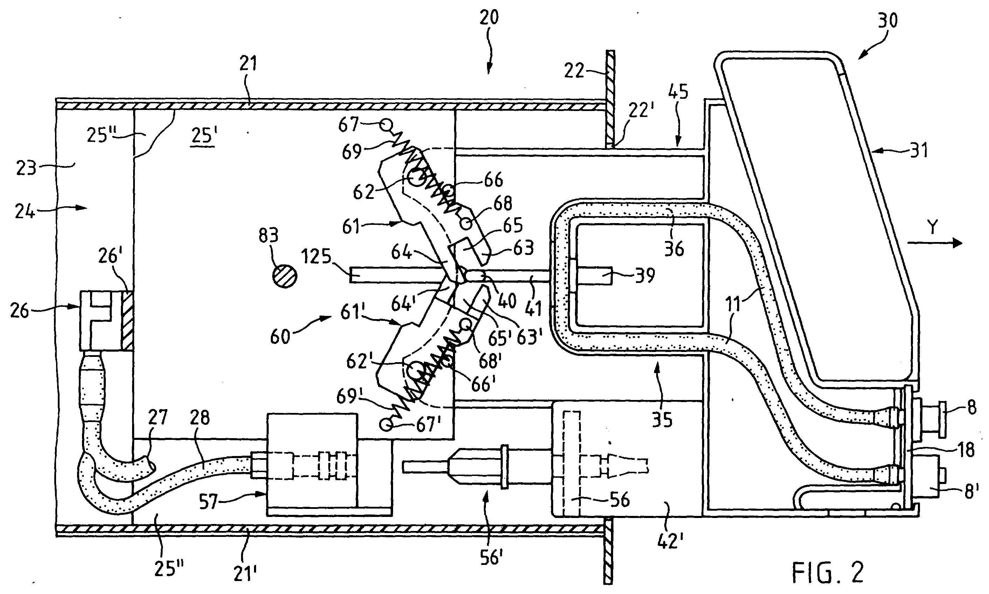

In dem Trägerelement 20 sind mehrere Funktionselemente angeordnet, mit welchen das Einschubelement 30 in eingeschobenem Zustand in Wirkverbindung steht. Die in verschiedenen Funktionsebenen im Trägerelement 20 angeordneten Funktionselemente sowie eine entsprechend ausgebildete Einzugs- und Verriegelungsmechanik 60 (Fig.2) für das Einschubelement 30 werden nachstehend beschrieben.

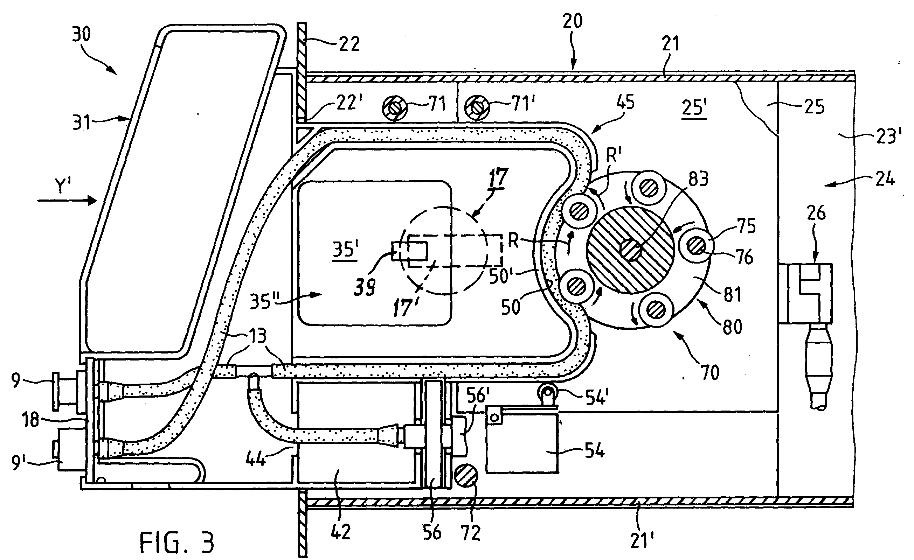

In Fig.2 ist ein in grösserem Massstab und im Schnitt dargestelltes Teilstück des Trägerelements 20 dargestellt. Das im wesentlichen kastenförmig ausgebildete Trägerelement 20 hat eine obere und eine untere Bodenplatte 21 und 21', zwei Seitenwände 23 und 23' (Fig.2,3), eine Stirnplatte 22 sowie eine nicht dargestellte Rückwand, wobei die Teile 21,21' und 23,23' sowie 22 im wesentlichen die zur Aufnahme der einzelnen Funktionselemente ausgebildete Kammer 24 bilden. In der Stirnplatte 22 ist die Öffnung 22' vorgesehen, durch welche das beispielsweise kassettenförmig ausgebildete Einschubelement 30 in die Kammer 24 einschiebbar ist. Zwischen den beiden Bodenplatten 21 und 21' ist, wie in Fig.3 dargestellt, eine erste Zwischenwand 25 angeordnet, welche mit nicht dargestellten Mitteln in dem Trägerelement 20 befestigt ist. An der ersten Zwischenwand 25 ist ein nicht dargestelltes Antriebsaggregat für eine Pumpeneinheit 70 (Fig.3) angeordnet. Weiterhin sind an der ersten Zwischenwand 25 ein Magnetventil 17 und ein davon betätigbares Quetschelement 17' und ein nicht dargestelltes Belüftungsventil sowie ein an eine Leitung 27 angeschlossenes Druckmessgerät 26 angeordnet. An die Leitung 21 ist ferner eine mit einem Anschlussgehäuse 57 (Fig.2) in Verbindung stehende Leitung 28 angeschlossen.

Die mit nicht dargestellten Mitteln an der ersten Zwischenwand 25 befestigten und schematisch dargestellten Funktionselemente 17,17' und 26 bilden zusammen mit der ersten Zwischenwand 25 eine erste Funktionsebene für das Einschubelement 30. Das Druckmessgerät 26 ist mit einer Halterung 26' an der ersten Zwischenwand 25 sowie an einer im Abstand dazu angeordneten zweiten Zwischenwand 25' (Fig.2) befestigt.

Fig.2 zeigt das in grösserem Massstab und im Schnitt dargestellte Trägerelement 20 sowie das in Pfeilrichtung Y teilweise aus der Kammer 24 herausgezogen dargestellte Einschubelement 30. Weiterhin erkennt man die zweite Zwischenwand 25', welche ebenfalls mit nicht dargestellten Mitteln in dem Trägerelement 20 befestigt ist. An der zweiten Zwischenwand 25' ist die Einzugs- und Verriegelungsmechanik 60 engeordnet, welche in der in Fig.2 dargestellten Stellung im wesentlichen ausser Eingriff des am Einschubelement 30 angeordneten Rastnockens 40 ist. An dem Einschubelement 30 ist das erste Tragteil 35 angeordnet, welches einen ersten Kanal 36 für eine erste Schlauchleitung 11 der Irrigationseinheit 10 (Fig.1) aufweist. Weiterhin erkennt man das etwa als ebene Platte ausgebildete und mit dem Rastnocken 40 versehene zweite Tragteil 45 für eine in Fig.3 dargestellte zweite Schlauchleitung 13 der Aspirationseinheit 12 (Fig.1). Das zweite Tragteil 45 wird später in Verbindung mit Fig.3 beschrieben. An der zweiten Zwischenwand 25' ist das Anschlussgehäuse 57 angeordnet und mit nicht dargestellten Mitteln befestigt. Das Anschlussgehäuse 57 ist auf der einen Seite über die Leitungen 28 und 27 mit dem Druckmessgerät 26 und an der anderen Seite zur Aufnahme eines Kupplungsstücks 56' ausgebildet. Das Kupplungsstuck 56' ist ein Teilstück des am Einschubelement 30 angeordneten Filters 56. Das Druckmessgerät 26 ist mit der Halterung 26' an der ersten sowie an der zweiten Zwischenwand 25,25' angeordnet und befestigt.

Die ebenfalls an der zweiten Zwischenwand 25' angeordnete Einzugs- und Verriegelungsmechanik 60 umfasst im wesentlichen, wie in Fig.2 dargestellt, zwei korrespondierend zueinander angeordnete und jeweils mit dem einen Ende über einen Zapfen 62 und 62' an der zweiten Zwischenwand 25' gelagerte Verriegelungshebel 61 und 61'. Die beiden Verriegelungshebel 61 und 61' sind gegen die Rückstellkraft damit in Wirkverbindung stehender Zugglieder 69 und 69' um die Achsen der beiden Zapfen 62 und 62' schwenkbar an der zweiten Zwischenwand 25' gelagert. An dem anderen Ende ist der einzelne Verriegelungshebel 61 und 61' jeweils durch eine Ausnehmung 65 und 65' sowie zwei im Abstand zueinander angeordnete Stege 63 und 64 beziehungsweise 63' und 64' zum Umgreifen des am Einschubelement 30 angeordneten Rastnockens 40 gabelförmig ausgebildet. Die beiden Zugglieder 69,69' sind mit dem einen Ende jeweils an einem an der zweiten Zwischenwand 25' angeordneten und befestigten Zapfen 67 und 67' gehalten und stehen mit dem anderen Ende über jeweils an den beiden Verriegelungshebeln 61, 61' angeordnete und befestigte Zapfen 68 und 68' in Wirkverbindung. Die beiden Zugglieder 69 und 69' sind beispielsweise als Schraubenfedern ausgebildet und an beiden Enden mit angeformten, die Zapfen 67,67' und 68,68' umgreifenden Bogenteilen (nicht bezeichnet) versehen.

Beim Einführen des Einschubelements 30 in das Trägerelement 20 gemäss Pfeilrichtung Y' (Fig.1) wird der am Einschubelement 30 angeformte Rastnocken 40 gegen die einen Gabelteile 64,64' der beiden Verriegelungshebel 61,61' gedrückt. Hierbei werden die Verriegelungshebel 61,61' um die beiden Zapfen 62, 62' und dabei gleichzeitig die beiden Schraubenfedern 69,69' um die Zapfen 67,67' verschwenkt. Bei diesem Vorgang werden die beiden Schraubenfedern 69,69' gespannt. Beim Erreichen und Überschreiten einer nicht dargestellten Totpunktstellung der beiden Verriegelungshebel 61,61' wird durch die Rückstellkraft der in dieser Position vorgespannten Schraubenfedern 69,69' das Einschubelement 30 schnappartig in die Kammer 24 des Trägerelements 20 gezogen. Bei diesem Bewegungsablauf wird das Einschubelement 30 mit der daran ungeordneten zweiten Schlauchleitung 13 (Fig.3) mit der Pumpeneinheit 70 und das Kupplungsstück 56' mit dem Anschlussgehäuse 57 und die erste Schlauchleitung 11 (Fig.2) mit dem nicht dargestellten Magnetventil in Eingriff gebracht.

Beim Herausziehen des Einschubelements 30 in Pfeilrichtung Y (Fig.2) drückt der Rastnocken 40 gegen die anderen Gabelteile 63,63' der beiden Verriegelungshebel 61,61', so dass diese mit den beiden Schraubenfedern 69,69' verschwenkt werden. Die Schwenkbewegung der beiden Verriegelungshebel 61,61' wird durch zwei entsprechend zugeordnete und in der zweiten Zwischenwand 25' befestigte Zapfen 66 und 66', begrenzt. In der Endstellung (Fig.2) der beiden Verriegelungshebel 61,61' ist der Rastnocken 40 des Einschubelements 30 ausser Eingriff der beiden Verriegelungshebel 61 und 61', so dass das Einschubelement 30 gemäss Pfeilrichtung Y aus der Kammer 24 des Trägerelements 20 beziehungsweise aus einer nicht dargestellten Kammer des Gehäuses 1 herausgezogen werden kann.

Beim Einführen des Einschubelements 30 in das Trägerelement 20 gemäss Pfeilrichtung Y' wird, wie in Fig.3 dargestellt, das Einschubelement 30 mit dem Rastnocken 40 vorzugsweise in einem in der zweiten Zwischenwand 25' angeordneten in Einschubrichtung orientierten Schlitz 125 einerseits in bezug auf die Einzugs- und Verriegelungsmechanik 60 und andererseits in bezug auf die Pumpeneinheit 70 geführt. Das Einschubelement 30 wird dabei mit dem mit der zweiten Schlauchleitung 13 versehenen zweiten Tragteil 45, wie in Fig.3 dargestellt, mit der an einer dritten Zwischenwand 25'' angeordneten Pumpeneinheit 70 exakt in Eingriff gebracht. An der dritten Zwischenwand 25'' können zur Führung des Einschubelements 30 weiterhin im Abstand zueinander angeordnete Rollen 71,71' sowie Anschläge 72 vorgesehen werden, durch welche ein Verkanten des Einschubelements 30 im Trägerelement 20 verhindert wird. An der dritten Zwischenwand 25'' ist weiterhin mit nicht dargestellten Mitteln ein mit einem Tastorgan 54' versehener Schalter 54 befestigt. Von dem mit einem Tastorgan 54' versehenen Schalter 34 wird für die Funktionsbereitschaft in der Endstellung ein Signal an die Pumpeneinheit 70 weitergegeben. Durch das Signal wird die Pumpeneinheit 70 betätigt.

Die über eine Antriebswelle 83 (Fig.2,3) mit dem nicht dargestellten Antriebsaggregat in Wirkverbindung stehende Pumpeneinheit 70 umfasst, wie in Fig.3 dargestellt, ein in Pfeilrichtung R angetriebenes Pumpenrad 80. An dem Pumpenrad 80 sind in Umfangsrichtung im Abstand zueinander angeordnete Rollen 75 gelagert. Im dargestellten Ausführungsbeispiel sind an dem Pumpenrad 80 fünf am Umfang verteilt angeordnete Rollen 75 vorgesehen. Hierdurch wird erreicht, dass in jeder Stellung des Pumpenrades 80 mindestens zwei Rollen 75 mit der am zweiten Tragteil 45 angeordneten zweiten Schlauchleitung 13 (Fig.3) in Eingriff stehen. Die Rollen 75 sind jeweils mit nicht dargestellten Mitteln um eine Achse 76 entgegen der Drehrichtung des Pumpenrades 80 in Pfeilrichtung R' angetrieben. Die entgegen der Pfeilrichtung R des Pumpenrades 80 in Pfeilrichtung R' angetriebenen Rollen 75 bewirken einen optimalen Funktionsablauf des peristaltisch arbeitenden Pumpenrades 80, ohne dass dabei auf die zweite Schlauchleitung 13, welche an der kreisbogenförmigen Kurvenbahn 50 des Tragteils 45 anliegt, eine die Schlauchleitung 13 streckende Zugwirkung übertragen wird.

In Fig.4 ist das Einschubelement 30 gemäss der in Fig.1 eingezeichneten Pfeilrichtung IV in Seitenansicht dargestellt. Das Einschubelement 30 umfasst im wesentlichen die beiden Tragteile 35 und 45 sowie das durch einen senkrechten Steg 34 begrenzt dazu angeordnete Griffstück 31. Bei dem dargestellten Ausführungsbeispiel hat das Griffstück 31 zur Stabilisierung zwei in parallelem Abstand zueinander angeordnete Leisten 32,32', eine dazwischen angeordnete erste Füllplatte 31' sowie eine zwischen dem senkrechten Steg 34 und der einen Leiste 32' angeordnete zweite Füllplatte 31''. An der Stirnseite des Griffstücks 31 ist weiterhin eine Ausnehmung 33 vorgesehen, welche zur Aufnahme der mit den Anschlussstutzen 8,8' und 9,9' versehenen Platte 18 (Fig.1 bis 3) ausgebildet ist.

Das an dem senkrechten Steg 34 angeformte erste Tragteil 35 hat beispielsweise einen durch eine Bodenplatte 35' begrenzten Hohlraum 35'' (Fig.5), eine die äussere Kontur der Bodenplatte 35' begrenzende erste Stegleiste 38 sowie eine in parallelem Abstand dazu angeordnete und die innere Kontur begrenzende zweite Stegleiste 37. Die beiden in bezug auf die Bodenplatte 35' abstehend ausgebildeten Stegleisten 37 und 38 bilden einen ersten Kanal 36 für die in Figur 2 dargestellte erste Schlauchleitung 11. Auf der der Kurvenhahn 50 zugewandten Seite ist an der Bodenplatte 45' des zweiten Tragteils 45 der als Mitnehmer ausgebildete Rastnocken 40 sowie eine sich daran anschliessende und bis zum ersten Tragteil 35 reichende Führungsrippe 41 angeordnet. In den beiden Stegleisten 37 und 38 ist auf der der Kurvenbahn 50 zugewandten Seite für das mit der ersten Schlauchleitung 11 (Fig.2) in Eingriff bringbare Quetschelement 17' (Fig.3) je eine Ausnehmung 37',38' und in der Bodenplatte 35' eine Öffnung 39 vorgesehen. Weiterhin ist der senkrechte Steg 34 im Bereich des Kanals 36 mit Ausnehmungen 36',36'' versehen, durch welche die erste Schlauchleitung 11 (Fig.2) hindurchführbar ist. Seitlich an der Bodenplatte 45' des zweiten Tragteils 45 ist ein durch eine Wand 42' begrenzter Raum 42 vorgesehen, welcher zur Aufnahme des mit dem Kupplungsstück 56' und dem Filter 56 (Fig.3) in Verbindung stehenden Leitungsstücks der zweiten Schlauchleitung 13 ausgebildet ist.

In Fig.5 ist das Einschubelement 30 gemäss der in Fig.1 eingezeichneten Pfeilrichtung V in Seitenansicht dargestellt und man erkennt das zweite Tragteil 45 sowie das durch einen senkrechten Steg 34' begrenzt dazu angeordnete und vorstehend in Verbindung mit Fig.4 beschriebene Griffstück 31. Das an dem senkrechten Steg 34' angeformte zweite Tragteil 45 umfasst die Bodenplatte 45', eine die äussere Kontur begrenzende erste Stegleiste 47,47' sowie eine in parallelem Abstand dazu angeordnete und die innere Kontur eines zweiten Kanals 46 begrenzende zweite Stegleiste 52,52'. An die beiden äusseren Stegleisten 47,47' ist je ein Bogenstück 48,48' und an den beiden inneren Stegleisten 52,52' je ein Bogenstück 51,51' angeformt. Der zweite Kanal 46 ist auf der der kreisbogenförmigen Kurvenbahn 50 zugewandten Seite mit Ausnehmungen 49,49' und auf der dem Steg 34' zugewandten Seite mit Ausnehmungen 46',46'' versehen. Durch die an der Bodenplatte 45' angeformten und korrespondierend zueinander angeordneten Bogenstücke 48,51 und 48',51' wird die eingelegte zweite Schlauchleitung 13 ohne Knickstellen der kreisbogenförmigen Kurvenbahn 50 zugeführt.

Die beiden in bezug auf die Bodenplatte 45' abstehenden Stegteile 47,47' und die Bogenstücke 48,48' sowie die Stegteile 52,52' und die Bogenstücke 51,51' sowie die Bogenleiste 50' der Kurvenbahn 50 bilden zusammen den zweiten Kanal 46 für die in Figur 3 dargestellte zweite Schlauchleitung 13. Weiterhin erkennt man den seitlich an der Bodenplatte 45' des zweiten Tragteils 45 angeordneten Raum 42. Auf der dem Steg 34' gegenüberliegenden Seite ist neben dem Raum 42 eine durch zwei beabstandete Seitenwände 53,53' gebildete Aufnahmekammer 55 angeordnet. In dem Steg 34' ist eine Ausnehmung 44 und in den Seitenwänden 53,53' jeweils eine weitere Ausnehmung 44' und 44'' angeordnet. Die beiden Ausnehmungen 44' und 44'' dienen zur Lagerung des Filters 56 (Fig.3) und die Ausnehmung 44 zur Durchführung eines mit dem Filter 56 in Verbindung stehenden Teilstücks der Schlauchleitung 13.

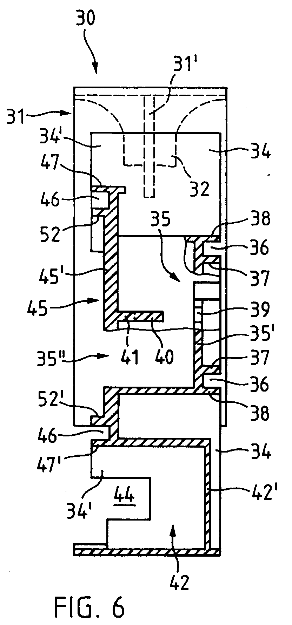

Fig.6 zeigt das gemäss der Linie VI-VI in Fig.4 im Schnitt dargestellte Einschubelement 30, und man erkennt die beiden parallel und quer zur Einschubrichtung Y' (Fig.1) in parallelem Abstand zueinander angeordneten Tragteile 35 und 45. An der Bodenplatte 35' sind die im Abstand zueinander angeordneten Stege 37 und 38 angeordnet, welche den umlaufenden ersten Kanal 36 bilden. Das erste Tragteil 35 bildet einen zum zweiten Tragteil 45 hin offenen Hohlraum 35''. An der Bodenplatte 45' des zweiten Tragteils 45 erkennt man die den zweiten Kanal 46 bildenden Stege 47,52 und 47',52'. Im unteren Bereich ist der durch die Wand 42' begrenzte Raum 42 mit dem Steg 34' angeordneten Ausnehmung 44 dargestellt.

Die vorstehend in Verbindung alt den Figuren 4 bis 6 beschriebene und zur Aufnahme der beiden Schlauchleitungen 11 und 13 ausgebildete Einschubelement 30 ist vorzugsweise aus geeignetem Kunststoff hergestellt. Das Einschubelement 30 ist als eine schnell aus der Kammer 24 der Einrichtung 100 herausziehbare und wieder hineinschiebbare Baueinheit ausgebildet.

## Patentansprüche

1. Einschubelement mit einer ersten und einer zweiten Schlauchleitung, welches in das Gehäuse einer ophthalmologischen Einrichtung (100) einschiebbar sowie durch eine Verriegelungsmechanik (60) derart gehalten und angeordnet ist, dass die erste Schlauchleitung (11) mit einem in dem Gehäuse (20) angeordneten Quetschelement wirkverbunden und die zweite Schlauchleitung (13) im Bereich einer Kurvenbahn mit einem ebenfalls in dem Gehäuse (20) angeordneten und um eine Achse (83) drehbar angetriebenen Pumpenrad (80) peristaltisch zusammenwirkend in Eingriff bringbar ist, **gekennzeichnet durch** ein an einem Griffstück (31) in Einschubrichtung orientiertes erstes Tragteil (35) mit daran angeordnetem ersten Kanal (36) für die erste Schlauchleitung (11) sowie ein quer zur Einschubrichtung im Abstand zu dem ersten Tragteil (35) angeordnetes und im wesentlichen als eine senkrecht zur Einschubrichtung orientierte Bodenplatte (45') ausgebildetes zweites Tragteil (45), welches an der dem ersten Tragteil (35) zugewandten Seite mit der Verriegelungsmechanik (60) in Eingriff bringbare Mittel aufweist und an der der ersten Schlauchleitung (11) abgewandten Seite mit einem zweiten Kanal (46) für die zweite Schlauchleitung (13) versehen ist, wobei die zweite Schlauchleitung (13) an einer stirnseitig an dem plattenförmigen Tragteil (45) angeordneten und analog der Kurvenbahn (50) ausgebildeten Bogenleiste (50') anliegend mit dem Pumpenrad (80) in Eingriff bringbar ist.

2. Einschubelement nach Anspruch 1, **dadurch gekennzeichnet**, dass das erste Tragteil (35) für die erste Schlauchleitung (11) sowie das etwa plattenförmig ausgebildete zweite Tragteil (45) für die zweite Schlauchleitung (13) an einem etwa senkrecht dazu orientierten Steg (34) des Griffstücks (31) angeordnet, vorzugsweise angeformt sind.

3. Einschubelement nach den Ansprüchen 1 und 2, **dadurch gekennzeichnet**, dass das quer zur Einschubrichtung im Abstand zu dem zweiten Tragteil (45) angeordnete erste Tragteil (35) mit einem Hohlraum (35'') etwa gehäuseförmig ausgebildet ist und eine Bodenplatte (35') aufweist, an dessen Aussenseite der etwa im Profilquerschnitt U-förmig ausgebildete sowie mit parallel und quer dazu angeordneten Stegleisten (37,38) versehene erste Kanal (36) für die erste Schlauchleitung (11) angeordnet ist.

4. Einschubelement nach Anspruch 3, **dadurch gekennzeichnet**, dass die Stegleisten (37,38) des ersten Kanals (36) an dem dem senkrechten Steg (34) des Griffstücks (31) zugewandten Ende mit einer Ausnehmung (36';36'') für die erste Schlauchleitung (11) und an der der Kurvenbahn (50) zugewandten Seite durch Ausnehmungen (37',38') unterbrochen sind und im Bereich derselben eine die Bodenplatte (35') durchdringende und mit dem Hohlraum (35'') in Verbindung stehende Ausnehmung (39) für das Quetschelement (17') vorgesehen ist.

5. Einschubelement nach den Ansprüchen 1 und 2, **dadurch gekennzeichnet**, dass der für die zweite Schlauchleitung (13) an der Bodenplatte (45') des zweiten Tragteils (45) vorgesehene zweite Kanal (46) im Profilquerschnitt etwa U-förmig ausgebildet ist und je zwei ausgehend von dem senkrechten Steg 34 des Griffstücks (31) in parallelem Abstand zueinander in Einschubrichtung orientierte Stegleisten (47,52;47',52') aufweist, welche am vorderen Ende jeweils mit einem an die Kurvenbahn (50) anschliessenden Bogenstück (48,51;48',51') versehen sind, wobei das einzelne Bogenstück (48,51;48',51') zum Durchführen des an der Kurvenbahn (50) anliegenden Schlauchteilstücks der zweiten Schlauchleitung (13) eine Ausnehmung (49,49') aufweist.

6. Einschubelement nach Anspruch 5, **dadurch gekennzeichnet**, dass die beiden in parallelem Abstand zueinander angeordneten inneren Stegleisten (52,52') des zweiten Kanals (46) mittels der Bogenstücke (51,51') an die Bogenleiste (50') der Kurvenbahn (50) angeordnet, vorzugsweise angeformt sind.

7. Einschubelement nach den Ansprüchen 5 und 6, **dadurch gekennzeichnet**, dass die Stegleisten (47,52;47',52') des zweiten Kanals (46) an dem dem senkrechten Steg (34) des Griffstücks (31) zugewandten Ende zur Durchführung der zweiten Schlauchleitung (13) mit einer Ausnehmung (46',46'') versehen sind.

8. Einschubelement nach Anspruch 1, **dadurch gekennzeichnet**, dass an der Bodenplatte (45') des zweiten Tragteils (45) ein mit der Verriegelungsmechanik (60) in Eingriff bringbarer Rastnocken (40) sowie eine daran anschliessende und bis zu dem ersten Tragteil (35) reichende Führungsrippe (41) angeordnet ist.

9. Einschubelement nach Anspruch 1, **dadurch gekennzeichnet**, dass an dem zweiten Tragteil (45) eine Aufnahmekammer (55) für einen mit der zweiten Schlauchleitung (13) in Verbindung stehenden Filter (56) angeordnet ist.

10. Einschubelement nach Anspruch 1, **dadurch gekennzeichnet**, dass die beiden in den Kanälen (36,46) angeordneten Schlauchleitungen (11,13) durch eine Zwischenwand (31'') des Griffstücks (31) voneinander getrennt und mit den Endstücken jeweils an einer stirnseitig am Griffstück (31) angeordneten und mit Anschlussstutzen (8,8'; 9,9') versehenen Platte (18) angeschlossen sind.
